# EUROPÄISCHE PATENTSCHRIFT

(11) **EP 3 329 747 B1**
(45) Veröffentlichungstag und Bekanntmachung des Hinweises auf die Patenterteilung: **23.09.2020**
(21) Anmeldenummer: 16732268.4
(22) Anmeldetag: 22.06.2016
(51) Int. Cl.: H05H 1/24, A61L 2/14

(54) **ELEKTRODENANORDNUNG UND PLASMABEHANDLUNGSVORRICHTUNG FÜR EINE OBERFLÄCHENBEHANDLUNG EINES KÖRPERS**
ELECTRODE ARRANGEMENT AND PLASMA-TREATMENT APPARATUS FOR SURFACE-TREATING A BODY
AGENCEMENT D'ÉLECTRODES ET DISPOSITIF DE TRAITEMENT AU PLASMA POUR UN TRAITEMENT DE SURFACE D'UN CORPS

(30) Priorität: 27.07.2015 DE 102015112200
(43) Veröffentlichungstag der Anmeldung: 06.06.2018
(73) Patentinhaber: Hochschule für Angewandte Wissenschaft und Kunst Hildesheim/Holzminden/Göttingen, 31134 Hildesheim (DE)
(72) Erfinder: VIÖL, Wolfgang, 37139 Adelebsen (DE); WIENEKE, Stephan, 37079 Göttingen (DE); TEN BOSCH, Lars, 31174 Schellerten OT Ottbergen (DE); KÖHLER, Robert, 37085 Göttingen (DE)
(74) Vertreter: REHBERG HÜPPE + PARTNER
(86) Internationale Anmeldenummer: PCT/EP2016/064440
(87) Internationale Veröffentlichungsnummer: WO 2017/016761

(56) Entgegenhaltungen:
- EP-A2- 1 215 947
- DE-A1-102009 045 498
- DE-A1-102012 103 362
- DE-A1-102013 000 440
- US-A1- 2005 067 934

## Beschreibung

### TECHNISCHES GEBIET DER ERFINDUNG

Die Erfindung bezieht sich auf eine Elektrodenanordnung für eine Oberflächenbehandlung eines Körpers mit einem physikalischen Plasma, die die Merkmale des Oberbegriffs des unabhängigen Patentanspruchs 1 aufweist, sowie auf eine Plasmabehandlungsvorrichtung mit einem Wechselhochspannungsgenerator und einer solchen Elektrodenanordnung.

Die Behandlung einer Oberfläche mit einem physikalischen Plasma kann aus unterschiedlichen Gründen erfolgen. Die jeweilige Oberfläche kann zum Zwecke einer besseren Haftung einer anschließenden Beschichtung physikalisch aktiviert werden, oder Keime oder Schädlinge an der Oberfläche können abgetötet werden. Entsprechend kann der Körper, dessen Oberfläche behandelt wird, ganz unterschiedlicher Natur sein. Es kann sich insbesondere um einen leblosen oder einen lebenden Körper handeln.

Die vorliegende Erfindung bezieht sich nicht auf eine Plasmabehandlung mit jeglichem physikalischen Plasma, sondern auf eine Plasmabehandlung mit einem sogenannten kalten Plasma, dessen Gastemperatur unter 100 °C und vorzugsweise in der Nähe der Umgebungstemperatur bleibt, so dass die Effekte des Plasmas zumindest weit überwiegend nicht thermischer Natur sind. Um ein kaltes Plasma durch eine Gasentladung zu erzeugen, ist die Gasentladung dielektrisch zu behindern, um den durch die Gasentladung fließenden Strom zu begrenzen. Eine dielektrische Barriere zur dielektrischen Behinderung der Gasentladung kann entweder vor einer an die Entladungsstrecke angrenzenden Elektrode, an der eine die Gasentladung hervorrufende Wechselhochspannung anliegt, oder auch zwischen der Elektrode und einem die Wechselhochspannung erzeugenden Wechselhochspannungsgenerator angeordnet werden.

### STAND DER TECHNIK

Eine Elektrodenanordnung mit den Merkmalen des Oberbegriffs des unabhängigen Patentanspruchs 1 und eine entsprechende Plasmabehandlungsvorrichtung sind aus der DE 10 2011 050 631 A1 bekannt. An einen Ausgang einer Wechselhochspannungsquelle sind mehrere gestreckte Elektrodenkörper aus Metall einzeln kapazitiv angekoppelt. Mit ihren Haupterstreckungsrichtungen verlaufen die gestreckten Elektrodenkörper parallel zueinander, und sie liegen so in einer Ebene, dass ihre distalen Enden auf einer Linie mit stetigem Verlauf angeordnet sind. Wenn mit der Wechselhochspannungsquelle eine Wechselhochspannung an die Elektrodenkörper angelegt wird, können gegenüber einem geerdeten Objekt elektrische Ladungen hervorgerufen werden, die insbesondere von den distalen Enden der Elektrodenkörper ausgehen. Nach der DE 10 2011 050 631 A1 kann noch eine zweite Reihe von gestreckten Elektrodenkörpern vorgesehen sein, die an den anderen Anschluss der Wechselhochspannungsquelle angeschlossen ist, so dass die Wechselhochspannungsquelle die Wechselhochspannung zwischen den beiden V-förmig zueinander angeordneten Reihen der Elektrodenkörper hervorruft. Hierdurch werden auf den Bereich der wechselweise aufeinanderfolgenden distalen Enden der beiden Reihen konzentrierte elektrische Entladungen hervorgerufen. Auf diese Weise können beispielsweise Haarläuse und deren Vorformen abgetötet werden, wenn diese in den Bereich der Entladungsstrecken zwischen die distalen Enden der Elektrodenkörper geraten.

Eine Elektrodenanordnung mit den Merkmalen des Obergriffs des Anspruchs 1 und eine entsprechende Plasmabehandlungsvorrichtung sind auch aus der DE 10 2009 045 498 D1 bekannt.

Für eine schnelle großflächige Oberflächenbehandlung eines Körpers sind die bekannten Elektrodenanordnungen und die bekannten Plasmabehandlungsvorrichtungen nicht geeignet. Aus der DE 10 2009 060 627 A1 ist eine Elektrodenanordnung für eine dielektrisch behinderte Plasmabehandlung einer als Gegenelektrode verwendeten unregelmäßig dreidimensional geformten Oberfläche eines elektrisch leitenden Körpers bekannt. Die Elektrodenanordnung weist eine flächige Elektrode und ein Dielektrikum auf, das zur Anordnung mit einem definierten Abstand zu der zu behandelnden Oberfläche zur Ausbildung eines kalten Plasmas ausgebildet ist. Das Dielektrikum ist durch ein flexibles flächiges Material gebildet, das auf seiner zu der zu behandelnden Oberfläche zeigenden Seite mit einer Struktur versehen ist, um Luftführungsbereiche auszubilden, wenn das Dielektrikum auf der zu behandelnden Oberfläche aufliegt. Die flächige Elektrode ist flexibel ausgebildet und am Dielektrikum so befestigt, dass eine Schicht des Dielektrikums die Elektrode von der zu behandelnden Oberfläche abschirmt. Durch die Anlagebereiche des Dielektrikums an der zu behandelnden Oberfläche werden wesentliche Teile der Oberfläche mit dieser bekannten Elektrodenanordnung nicht behandelt.

Aus der DE 10 2007 030 915 A1 ist eine Plasmabehandlungsvorrichtung zur Behandlung von Oberflächen mit einem mittels einer Elektrode über ein Feststoff-Dielektrikum durch eine dielektrisch behinderte Gasentladung erzeugten Plasma bekannt. Dabei weist das Feststoff-Dielektrikum eine flexible Wirkoberfläche auf, die während der Behandlung unmittelbar an das Plasma angrenzt. Dazu kann das Dielektrikum durch flexible Hohlfasern gebildet sein, in denen die Elektrode angeordnet ist. Die Hohlfasern werden parallel zu der zu behandelnden Oberfläche angeordnet, insbesondere im Verbund mit Stützelementen in Form von Fasern, die mit den Hohlfasern ein gewebeartiges Element bilden. Bereits in der von derselben Anmelderin stammenden DE 10 2009 060 627 A1 wird darauf hingewiesen, dass die aus der DE 10 2007 030 915 A1 bekannte Elektrodenanordnung aufwändig in der Herstellung ist.

Aus der US 6,494,881 B1 sind eine Vorrichtung und ein Verfahren zur elektro-chirurgischen Gewebeentfernung mit einer bereichsweise isolierten Elektrode bekannt. Andere Bereiche der an eine Wechselhochspannungsquelle angeschlossenen Elektrode sind nicht elektrisch isoliert. Eine von der Wechselhochspannungsquelle erzeugte Wechselhochspannung liegt zwischen dieser Elektrode und einer weiteren, ebenfalls nur bereichsweise isolierten Elektrode an. Mit der bekannten Vorrichtung wird Gewebe elektro-chirurgisch entfernt, dass zwischen die beiden U-förmig gebogenen Elektroden gelangt.

Aus der US 8,979,838 B2 ist eine elektro-chirurgische Einrichtung mit zwei in entgegengesetzten Richtungen abgewinkelten U-förmig gebogenen Elektroden aus elektrisch leitfähigem Material bekannt. Zwischen den beiden Elektroden wird eine von einer Wechselhochspannungsquelle erzeugte Wechselhochspannung angelegt.

Aus der DE 10 2012 103 362 A1 ist ein Plasmabehandlungsgerät insbesondere zur Behandlung des menschlichen Körpers bekannt, das stiftförmig ausgebildet ist, wobei eine erste Elektrode in einem Stiftteil vorgesehen ist und das Stiftteil im Bereich der Elektrode geerdet ist. Die erste Elektrode ist an der Spitze des Stiftteils bogenförmig umgebogen, so dass ihr distales Ende zurück in den Stiftteil verläuft. Die Elektrode ist mit einem Dielektrikum umhüllt. An der Elektrode wird eine von einer Wechselhochspannungsquelle gegenüber Erde erzeugte Wechselhochspannung angelegt. Eine zweite, ringförmige Elektrode auf Erdpotential kann um einen mit dem Plasmabehandlungsgerät zu behandelnden Bereich herum auf eine Hautfläche aufgelegt werden.

Aus der DE 691 30 110 T2 ist ein Generator zum Erzeugen aktiver Spezies bekannt, der dielektrisch beschichtete kleinkalibrige Elektroden umfasst. In einen Reaktorraum, der von Gas durchströmt wird, ragen dielektrisch beschichtete Elektrodendrähte hinein, die an unterschiedliche Hochspannungsanschlüsse einer Wechselhochspannungsquelle angeschlossen sind, und zwischen denen eine von der Wechselhochspannungsquelle erzeugte Wechselhochspannung angelegt wird. Dadurch werden in dem durch den Generator strömenden Gas dielektrisch behinderte Entladungen zwischen den Elektrodendrähten hervorgerufen.

Aus der DE 10 2009 028 190 A1 ist eine Vorrichtung zur Erzeugung eines nichtthermischen Atmosphärendruck-Plasmas bekannt. Die Vorrichtung umfasst ein im Bereich des austretenden Plasmas als geerdete Elektrode dienendes Metallgehäuse, in dem ein Hochfrequenzgenerator, eine Hochfrequenzresonanzspule mit einem für die Hochfrequenz geeigneten geschlossenen Ferritkern, ein als Gasdüse dienender Isolierkörper sowie ein in dem Isolierkörper gelagerte Hochspannungselektrode in der Weise angeordnet sind, dass die Hochspannungselektrode von Prozessgas durchströmt wird.

Aus der WO 2009/074546 A1 sind ein Verfahren und eine Vorrichtung zur Behandlung von Oberflächen mit einem bei Atmosphärendruck erzeugten Plasma bekannt. Die Vorrichtung ist als tragbares Handgerät ausgebildet und umfasst eine Plasmadüse zur Erzeugung eines Plasmastrahls, die eine Düsenöffnung und wenigstens ein stromaufwärts von der Düsenöffnung angeordnetes Elektroden- und Gegenelektroden-Paar umfasst, deren wirksame Elektrodenoberflächen jeweils eine dielektrische Beschichtung aufweisen. Die Elektrode und die Gegenelektrode definieren zwischen sich einen Arbeitsraum, in welchem ein Arbeitsgas mittels dielektrischer behinderter Gasentladung wenigstens teilweise ionisiert werden kann. Weiterhin umfasst die Vorrichtung in dem Handgerät einen Hochspannungsgenerator, der mit dem Elektroden- und Gegenelektronen-Paar elektrisch verbunden ist, ein Fördermittel, welches einen Gasfluss des Arbeitsgases von einer Arbeitsgasquelle in den Arbeitsraum und durch die Düsenöffnung erzeugt, wobei die Arbeitsgasquelle die Umgebungsluft ist, und eine netzunabhängige Energiequelle zur Versorgung des Hochspannungsgenerators und des Fördermittels.

### AUFGABE DER ERFINDUNG

Der Erfindung liegt die Aufgabe zugrunde, eine Elektrodenanordnung und eine mit einer solchen Elektrodenanordnung ausgestattete Plasmabehandlungsvorrichtung aufzuzeigen, die bei geringem Herstellungsaufwand eine schnelle großflächige Oberflächenbehandlung ermöglichen.

### LÖSUNG

Die Aufgabe der Erfindung wird durch eine Elektrodenanordnung mit den Merkmalen des unabhängigen Patentanspruchs 1 und eine Plasmabehandlungsvorrichtung mit den Merkmalen des nebengeordneten Patentanspruchs 14 gelöst. Bevorzugte Ausführungsformen der Elektrodenanordnung und der Plasmabehandlungsvorrichtung sind in den abhängigen Patentansprüchen definiert.

### BESCHREIBUNG DER ERFINDUNG

Die Erfindung betrifft eine Elektrodenanordnung für eine Oberflächenbehandlung eines Körpers mit einem physikalischen Plasma, die einen Hochspannungsanschluss zum Anschließen an einen Ausgang einer eine Wechselhochspannung erzeugenden Wechselhochspannungsquelle und mehrere an den Hochspannungsanschluss angeschlossene linienförmige Elektrodenkörper aufweist. Die Elektrodenkörper sind derart gemeinsam an den Hochspannungsanschluss angeschlossen, dass an allen Elektrodenkörpern dieselbe Wechselhochspannung anliegt, und sie sind mit einem Dielektrikum ummantelt und/oder einzeln kapazitiv an den Hochspannungsanschluss gekoppelt. Die Elektrodenkörper sind insofern linienförmig als dass sie sich jeweils im Wesentlichen längs einer Linie erstrecken, bei der es sich aber um keine Gerade handelt. Erfindungsgemäß umfassen die Elektrodenkörper vielmehr jeweils einen bogenförmigen Bereich, in dem der jeweilige linienförmige Elektrodenkörper seine Richtung um mindestens 90° ändert. Ein an die Elektrodenkörper angrenzender Behandlungsraum, in den ein Körper für eine Oberflächenbehandlung mit dem physikalischen Plasma einzubringen ist, befindet sich zumindest teilweise zwischen diesen bogenförmigen Bereichen der Elektrodenkörper. Wenn an dem Hochspannungsanschluss und damit an den Elektrodenkörpern eine Wechselhochspannung gegenüber Erde oder Masse anliegt und wenn der für die Oberflächenbehandlung vorgesehene Körper geerdet ist oder eine ausreichend große elektrische Kapazität aufweist, bilden sich zwischen den bogenförmigen Bereichen der Elektrodenkörper und dem zu behandelnden Körper Gasentladungen aus. Die Entladungsstrecken verteilen sich dabei längs der bogenförmigen Bereiche der linienförmigen Elektrodenkörper, weil die größten elektrischen Felder, die im Bereich der kleinsten Oberflächenkrümmungsradien der Elektrodenkörper auftreten, über die bogenförmigen Bereiche verteilt und nicht etwa an einer zu dem jeweiligen zu behandelnden Körper weisenden freien Spitze der Elektrodenkörper konzentriert sind. Die dem zu behandelnden Körper zugekehrten kleinsten Oberflächenkrümmungsradien sind diejenigen der Oberfläche des Querschnitts der linienförmigen Elektrodenkörper, der über den bogenförmigen Bereich jedes Elektrodenkörpers vorzugsweise konstant ist. Durch die Verteilung der Entladungsstrecken längs der bogenförmigen Elektrodenkörper liegt ein mit der erfindungsgemäßen Elektrodenanordnung durch dielektrisch behinderte Gasentladung erzeugtes physikalisches Plasma großflächig über der Oberfläche des zu behandelnden Körpers vor. So wird diese Oberfläche schnell vollständig behandelt.

Die Elektrodenkörper können in ihren bogenförmigen Bereichen insbesondere eine äußere Oberfläche mit einem abgerundeten oder kreisrunden Querschnitt aufweisen, dessen Oberflächenkrümmungsradius über den Umfang des jeweiligen Elektrodenkörpers zumindest im Wesentlichen gleich ist. Grundsätzlich können die Elektrodenkörper aber auch eine äußere Oberfläche beispielsweise mit einem sternförmigen Querschnitt aufweisen, um an den Spitzen von Zacken des Sterns gezielt Feldspitzen des elektrischen Felds gegenüber einem zu behandelnden Körper hervorzurufen, die aber dennoch längs der linienförmigen Elektrodenkörper verteilt sind.

Eine Krümmung, mit der der jeweilige linienförmige Elektrodenkörper in dem bogenförmigen Bereich seine Richtung ändert, kann grundsätzlich konstant sein, sie kann aber auch variieren. Typischerweise liegt der Krümmungsradius dieser Krümmung zwischen 3 mm und 300 mm. Oftmals liegt er zwischen 5 mm und 200 mm. Dieser Krümmungsradius ist in jedem Fall deutlich größer als der vorzugsweise über den bogenförmigen Bereich des jeweiligen linienförmigen Elektrodenkörpers gleichbleibende kleinste Oberflächenflächenkrümmungsradius. Dieser kleinste Oberflächenkrümmungsradius liegt typischerweise in einem Bereich zwischen 0,3 mm und 30 mm. In der Regel beträgt er zwischen 0,5 mm und 5 mm. Der Unterschied zwischen dem Krümmungsradius des bogenförmigen Bereichs und dem kleinsten Oberflächenkrümmungsradius des linienförmigen Elektrodenkörpers beträgt mindestens einen Faktor 3, häufig einen Faktor 5 oder 10 oder noch mehr.

Der jeweilige linienförmige Elektrodenkörper kann in dem bogenförmigen Bereich seine Richtung um deutlich mehr als 90° ändern, insbesondere um etwa 180°, d. h. mindestens 150° oder noch mehr. Der Elektrodenkörper wird damit zunehmend schleifen- oder schlaufenförmig. Insbesondere kann er sich dabei von und bis zu einem Sockel aus einem elektrisch isolierenden Material erstrecken. Das heißt, die Elektrodenkörper weisen keine freien Enden auf, sondern enden beidseitig in dem jeweiligen Sockel aus dem elektrisch isolierenden Material, in dem sie auch an Hochspannungsanschluss angeschlossen sein können. Jede Feldkonzentration an freien distalen Enden der Elektrodenkörper und eine damit verbundene nur punktuelle Ausbildung einer Gasentladung ausgehend von den distalen Enden der Elektrodenkörper wird damit vermieden.

Der Behandlungsraum, in dem eine Oberflächenbehandlung mit der erfindungsgemäßen Elektrodenanordnung erfolgt, kann sich zumindest teilweise zwischen zwei Ebenen befinden, in denen sich zwei einander benachbarte Elektrodenkörper mit ihren bogenförmigen Bereichen erstrecken. Dieser Behandlungsraum kann z. B. gegenüber einem blattförmigen Körper mit der Elektrodenanordnung verschoben werden, um die beiden einander gegenüberliegenden Oberflächen des blattförmigen Körpers mit dem physikalischen Plasma zu behandeln. So können beispielsweise von Parasiten oder Mikroorganismen befallene Blätter einer Pflanze mit dem physikalischen Plasma behandelt werden, um diese Parasiten bzw. Mikroorganismen abzutöten.

Der Behandlungsraum kann sich zumindest teilweise auch zwischen zwei Gruppen der Elektrodenkörper erstrecken, deren bogenförmige Bereiche sich mit einander entgegen gerichteten Krümmungsradien gegenüberliegen. Auch in diesem Fall ist es bei der erfindungsgemäßen Elektrodenanordnung so, dass alle Elektrodenkörper, d. h. die Elektrodenkörper beider Gruppen, an einen gemeinsamen Hochspannungsanschluss angeschlossen sind und dass die Gasentladungen dementsprechend nicht zwischen den beiden Gruppen von Elektrodenkörpern, sondern gegenüber einem in dem Behandlungsraum eingebrachten Körper hervorgerufen werden.

Die bogenförmigen Bereiche der Elektrodenkörper jeder Gruppe können in zueinander parallelen Ebenen verlaufen. Hiermit wird eine gleichmäßige und dichte Verteilung der Elektrodenkörper und der damit hervorgerufenen Gasentladungen bewirkt. Die Elektrodenkörper können sich dabei sowohl innerhalb einer Gruppe oder auch über die beiden Gruppen hinweg berühren, ohne dass dies der Funktion der Elektrodenanordnung einen Abbruch tut, weil an allen Elektrodenkörpern dieselbe Wechselhochspannung anliegt.

Zumindest in ihren bogenförmigen Bereichen, d. h. auch insgesamt, können die Elektrodenkörper gegen Rückstellkräfte verformbar sein. So kann ein zu behandelnder Körper, wenn er in den Behandlungsraum eingebracht wird, die den Behandlungsraum begrenzenden Elektrodenkörper so weit auseinanderschieben, dass er in den Behandlungsraum passt. Die Elektrodenkörper befinden sich damit in der unmittelbaren Nähe der Oberfläche des zu behandelnden Körpers.

Die Verformbarkeit der Elektrodenkörper kann gegen elastische Kräfte und/oder Gravitationskräfte gegeben sein, so dass sich die Elektrodenkörper selbsttätig zurückverformen, wenn beispielsweise der behandelte Körper wieder aus dem Behandlungsraum entfernt wird.

Die Elektrodenkörper können in einem den Behandlungsraum teilweise umschließenden Gehäuse angeordnet sein. Durch eine verbleibende Öffnung des Gehäuses kann der jeweils zu behandelnde Körper in den Behandlungsraum eingebracht werden. Das Gehäuse, in dem die Elektrodenkörper angeordnet sind, kann aus Sicherheitsgründen geerdet sein.

Eine erfindungsgemäße Plasmabehandlungsvorrichtung mit einem Wechselhochspannungsgenerator, der an einem Ausgang eine Wechselhochspannung gegenüber Erde erzeugt, weist eine an den Ausgang des Wechselhochspannungsgenerators angeschlossene erfindungsgemäße Elektrodenanordnung auf.

Der Wechselhochspannungsgenerator kann Wechselhochspannungen zwischen etwa 5 kV und 50 kV, insbesondere zwischen 5 kV und 30 kV erzeugen.

In diesen und anderen Ausführungsformen der erfindungsgemäßen Vorrichtung kann der Wechselhochspannungsgenerator die Wechselhochspannung mit einer Frequenz zwischen etwa 100 kHz und etwa 3 MHz oder zwischen 150 kHz und etwa 1 MHz oder zwischen 200 kHz und 600 kHz erzeugen. Durch diese Frequenzen der Wechselhochspannung wird eine erhöhte Betriebssicherheit der erfindungsgemäßen Vorrichtung gewährleistet, da auf Grund des Skineffektes bei einer versehentlichen Berührung von Teilen der Vorrichtung, an denen diese Wechselhochspannung anliegt, der resultierende elektrische Strom lediglich die Oberfläche des Körpers der berührenden Person entlang fließt und nicht tief in darunter liegendes Gewebe eindringt. Eine Gefährdung des Benutzers der erfindungsgemäßen Vorrichtung kann so ausgeschlossen werden.

Zusätzlich kann die Wechselhochspannung getaktet werden, um den thermischen Eintrag in das zu behandelnde Objekt und auch in den Körper einer mit der Wechselhochspannung in Berührung kommenden Person zu begrenzen. Dabei ist unter einer Taktung der Wechselhochspannung eine Unterbrechung der Wechselhochspannung nach jeweils einer Gruppe von Hochspannungspulsen wechselnder Polarität zu verstehen. Eine solche Gruppe kann z. B. einige bis einige zehn Hochspannungspulse wechselnder Polarität umfassen. Durch die Taktung der Wechselhochspannung kann trotz der Frequenz der Wechselhochspannung größer 100 kHz eine mittlere Pulswiederholungsfrequenz der Hochspannungspulse insbesondere in dem Bereich von 3 kHz bis 50 kHz eingestellt werden.

Bei Taktung der Wechselhochspannung kann durch Variation der Anzahl der Hochspannungspulse in jeder Gruppe und/oder einer Gruppenfolgefrequenz, mit der die einzelnen Gruppen der Hochspannungspulse aufeinander folgen, eine elektrische Leistung der durch die Wechselhochspannung hervorgerufenen Gasentladung auf einen gewünschten Wert, insbesondere einen gewünschten Wert pro Flächeneinheit der Oberfläche des behandelten Objekts, eingestellt werden. Diese Flächenleistungsdichte kann zwischen 0,1 bis 10 W/cm² liegen.

Die Gesamtausgangsleistung des Wechselhochspannungsgenerator kann in einem typischen Bereich von 5 W bis 500 W liegen.

Die Gruppenfolgefrequenz der Gruppen der Hochspannungspulse kann in einem Bereich jenseits der vom Menschen hörbaren Frequenzen liegen, d. h. mindestens 16 kHz betragen, so dass der Betrieb der Vorrichtung keine Geräusche mit der Wiederholungsfrequenz verursacht. Die Gruppenfolgefrequenz kann auch deutlich höher als die Grenze des hörbaren Bereichs liegen, d. h. mindestens 20 kHz oder mindestens 30 kHz betragen, so dass mit ihr keinerlei vom Menschen wahrnehmbare Schwingungsanregungen verbunden sind.

Vorteilhafte Weiterbildungen der Erfindung ergeben sich aus den Patentansprüchen, der Beschreibung und den Zeichnungen. Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen. Ohne dass hierdurch der Gegenstand der beigefügten Patentansprüche verändert wird, gilt hinsichtlich des Offenbarungsgehalts der ursprünglichen Anmeldungsunterlagen und des Patents Folgendes: weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einem Element die Rede ist, ist dies so zu verstehen, dass genau ein Element, zwei Elemente oder mehr Elemente vorhanden sind. Die in den Patentansprüchen angeführten Elemente können durch andere Elemente ergänzt werden oder die einzigen Elemente sein, aus denen das jeweilige Erzeugnis besteht.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: zeigt eine als Handgerät ausgebildete erfindungsgemäße Plasmabehandlungsvorrichtung mit zwei Elektrodenkörpern.
- **Fig. 2**: zeigt eine Ausführungsform einer erfindungsgemäßen Plasmabehandlungsvorrichtung mit einer Vielzahl von nebeneinander angeordneten bogenförmigen Elektrodenkörpern.
- **Fig. 3**: zeigt eine Ausführungsform der erfindungsgemäßen Plasmabehandlungsvorrichtung mit einer Vielzahl von nebeneinander angeordneten schlaufenförmigen Elektrodenkörpern; und
- **Fig. 4**: zeigt eine Ausführungsform der erfindungsgemäßen Plasmabehandlungsvorrichtung mit zwei einander gegenüberliegenden Gruppen von bogenförmigen Elektrodenkörpern.

### FIGURENBESCHREIBUNG

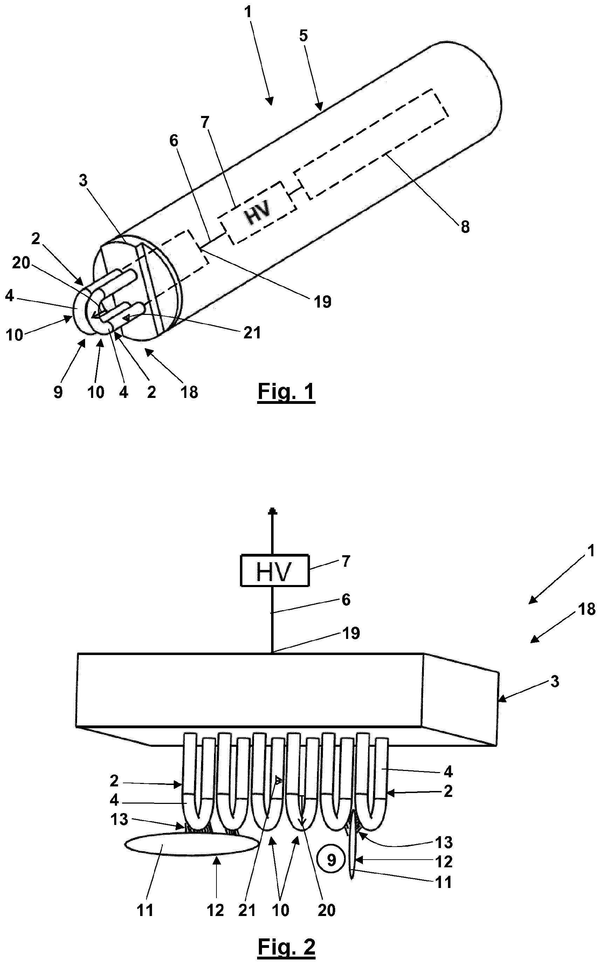

Die in **Fig. 1** dargestellte Plasmabehandlungsvorrichtung 1 umfasst eine Elektrodenanordnung 18 mit zwei bogenförmigen Elektrodenkörpern 2, die jeweils in einem Sockel 3 aus elektrisch isolierendem Material beginnen und enden und sich dabei in zwei parallel zueinander verlaufenden Ebenen zunächst geradlinig weg von dem Sockel 3, dann über einen 180° Bogen und zuletzt geradlinig zurück zu dem Sockel 3 erstrecken. Der Querschnitt der beiden jeweils mit einem Dielektrikum 4 ummantelten Elektrodenkörper 2 ist hier kreisrund. Ein Krümmungsradius 20, mit dem die Elektrodenkörper 2 ihre Richtung in ihren bogenförmigen Bereichen 10 ändern, ist hier etwa fünfmal so groß wie ihr kleinster Oberflächenkrümmungsradius 21 aufgrund ihres kreisrunden Querschnitts. Diesen minimalen Oberflächenkrümmungsradius 21 weisen die Elektrodenkörper 2 über ihre gesamten Erstreckungen von und bis zu dem Sockel 3 auf. Elektrisch sind die Elektrodenkörper 2 in einem geschlossenen Gehäuse 5 eines Handgeräts über einen Hochspannungsanschluss 19 gemeinsam an einen Ausgang 6 eines Wechselhochspannungsgenerators 7 angeschlossen, der aus einem Akkumulator 8 mit Energie versorgt wird. Der Wechselhochspannungsgenerator 7 erzeugt eine an dem Ausgang 6 anliegende Wechselhochspannung gegenüber seiner elektrischen Masse. Wenn ein Objekt, das elektrisch geerdet ist oder eine ausreichende elektrische Kapazität aufweist, in einen Behandlungsraum 9 zwischen den beiden Elektrodenkörpern 2 eingebracht wird, werden durch die an den Elektrodenkörper 2 anliegende Wechselhochspannung Gasentladungen gegenüber der Oberfläche des Körpers hervorgerufen, die zu einer Oberflächenbehandlung des Körpers mit einem physikalischen Plasma führen, das infolge der Gasentladungen entsteht. Die Gasentladungen verteilen sich über die gesamte Länge der Elektrodenkörper 2 außerhalb des Sockels 3, weil diese über ihre gesamte Länge hinweg ihren kleinsten Oberflächenkrümmungsradius 21 aufweisen, der das maximale elektrische Feld gegenüber dem zu behandelnden Körper bestimmt.

Bei der Ausführungsform der Plasmabehandlungsvorrichtung 1 gemäß **Fig. 2** ist eine Vielzahl, d. h. sind mehr als zwei Elektrodenkörper 2 in parallel zueinander verlaufenden Ebenen nebeneinander angeordnet, wobei sie jeweils in demselben Sockel 3 beginnen und enden und über denselben Hochspannungsanschluss 19 an denselben Ausgang 6 des Wechselhochspannungsgenerators 7 angeschlossen sind. Hier erstreckt sich der Behandlungsraum 9 zwischen den Elektrodenkörpern 2 und bis vor deren bogenförmige Bereiche 10. Illustriert sind zwei Körper 11, deren Oberflächen 12 in ihren den Elektrodenkörpern 2 gegenüberliegenden Bereichen infolge von sich dort ausbildenden Gasentladungen 13 mit einem physikalischen Plasma behandelt werden.

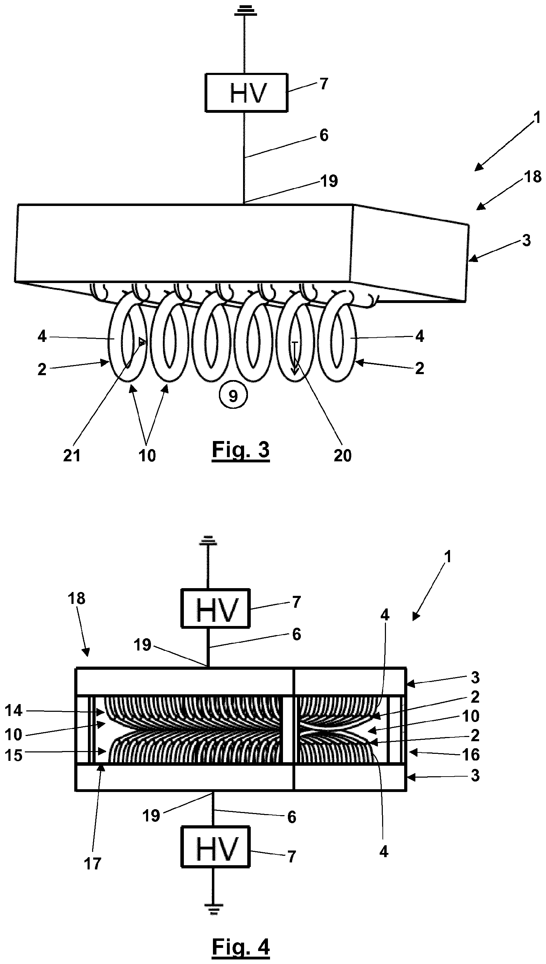

**Fig. 3** zeigt eine Abwandlung der Plasmabehandlungsvorrichtung 1 gemäß Fig. 2. Hier sind die Elektrodenkörper 2 jeweils schleifen- oder schlaufenförmig, wobei sie vollständig aus ihren bogenförmigen Bereichen 10 bestehen und über diese mit konstantem Krümmungsradius 20 ihre Richtungen über 360° ändern. Auch hier erstreckt sich der Behandlungsraum 9 vor den Elektrodenkörpern 2 und zwischen die Elektrodenkörper 2.

Die in **Fig. 4** dargestellte Plasmabehandlungsvorrichtung 1 weist zwei Gruppen 14 und 15 von Elektrodenkörpern 2 auf, die jeweils endseitig in einem Sockel 3 gelagert sind. Die Elektrodenkörper 2 der beiden Gruppen 14 und 15 liegen sich mit ihren bogenförmigen Bereichen 10 gegenüber, wobei die Elektrodenkörper 2 der beiden Gruppen, wie in Fig. 4 dargestellt, auch aneinander liegen können oder sogar ineinander eingreifen können. Der Behandlungsraum der Plasmabehandlungsvorrichtung 1 zwischen den Elektrodenkörpern 2 nimmt auch größere Körper für eine Oberflächenbehandlung auf, indem die Elektrodenkörper 2 einschließlich ihrer Ummantelung mit dem Dielektrikum 4 gegen Rückstellkräfte verformbar ausgebildet sind. So kann beispielsweise eine zu desinfizierende Hand in ein teilweise geöffnetes Gehäuse 16, in dem die beiden Gruppe 14 und 15 der Elektrodenkörper 2 angeordnet sind, durch eine seitliche Öffnung 17 hineingeschoben werden. Dauerhaft oder infolge des Hineinschiebens des Körpers in das Gehäuse 16 wird über die Hochspannungsanschlüsse 19 eine Wechselhochspannung angelegt, und zwar dieselbe an alle Elektrodenkörper 2, so dass eine resultierende Gasentladung nicht zwischen den Elektrodenkörpern 2, sondern zwischen der Oberfläche des zu behandelnden Körpers und den gegenüberliegenden oder anliegenden Elektrodenkörpern 2 hervorgerufen wird. Die Darstellung in Fig. 4 ist daher nicht so zu verstehen, dass zwei Wechselhochspannungsgeneratoren 7 unabhängige Wechselhochspannungen gegenüber Erde an Ihren Ausgängen 6 erzeugen. Die dichte Verteilung der an dem jeweiligen Körper angrenzenden Elektrodenkörper 2 und die über die Längserstreckungen der linienförmigen Elektrodenkörper 2 gleichbleibenden kleinsten Oberflächenkrümmungsradien der Elektrodenkörper 2 sorgen für eine großflächige Verteilung der sich zwischen dem jeweiligen Körper und den Elektrodenkörpern 2 ausbildenden Entladungsstrecken. So erfolgt eine schnelle vollflächige Oberflächenbehandlung des Körpers 2 mit dem durch die Gasentladung generierten physikalischen Plasma.

### BEZUGSZEICHENLISTE

- 1: Plasmabehandlungsvorrichtung
- 2: Elektrodenkörper
- 3: Sockel
- 4: Dielektrikum
- 5: Gehäuse
- 6: Ausgang
- 7: Wechselhochspannungsgenerator
- 8: Akkumulator
- 9: Behandlungsraum
- 10: bogenförmiger Bereich
- 11: Körper
- 12: Oberfläche
- 13: Gasentladung
- 14: Gruppe
- 15: Gruppe
- 16: Gehäuse
- 17: Öffnung
- 18: Elektrodenanordnung
- 19: Hochspannungsanschluss
- 20: Krümmungsradius
- 21: Oberflächenkrümmungsradius

## Patentansprüche

1. Elektrodenanordnung (18) für eine Oberflächenbehandlung eines Körpers (11) mit einem physikalischen Plasma mit:
- einem Hochspannungsanschluss (19) zum Anschließen an einen Ausgang (6) einer eine Wechselhochspannung erzeugenden Wechselhochspannungsquelle;
- mehreren linienförmigen Elektrodenkörpern (2), die derart gemeinsam an den Hochspannungsanschluss (19) angeschlossen sind, dass an allen Elektrodenkörpern (2) dieselbe Wechselhochspannung anliegt, und die mit einem Dielektrikum (4) ummantelt und/oder einzeln kapazitiv an den Hochspannungsanschluss (19) gekoppelt sind; und
- einem an die Elektrodenkörper (2) angrenzenden Behandlungsraum (9), in den der Körper (11) für die Oberflächenbehandlung mit dem physikalischen Plasma einbringbar ist; **dadurch gekennzeichnet, dass** die Elektrodenkörper (2) jeweils einen bogenförmigen Bereich (10) umfassen, in dem der jeweilige linienförmige Elektrodenkörper (2) seine Richtung um mindestens 90° ändert, wobei sich der Behandlungsraum (9) zumindest teilweise zwischen den bogenförmigen Bereichen (10) der Elektrodenkörper (2) befindet.

2. Elektrodenanordnung (18) nach Anspruch 1, **dadurch gekennzeichnet, dass** ein Krümmungsradius (20), mit dem der jeweilige linienförmige Elektrodenkörper (2) in dem bogenförmigen Bereich (10) seine Richtung ändert, zwischen 3 mm und 300 mm oder zwischen 5 mm und 200 mm beträgt.

3. Elektrodenanordnung (18) nach Anspruch 1 oder 2, **dadurch gekennzeichnet, dass** der jeweilige linienförmige Elektrodenkörper (2) in dem bogenförmigen Bereich (10) einen gleichbleibenden kleinsten Oberflächenkrümmungsradius (21) zwischen 0,3 mm und 30 mm oder zwischen 0,5 mm und 5 mm aufweist.

4. Elektrodenanordnung (18) nach einem der vorhergehenden Ansprüche, **dadurch gekennzeichnet, dass** die Elektrodenkörper (2) in den bogenförmigen Bereichen (10) eine äußere Oberfläche mit einem abgerundeten oder kreisrunden Querschnitt aufweisen.

5. Elektrodenanordnung (18) nach einem der vorhergehenden Ansprüche, **dadurch gekennzeichnet, dass** der jeweilige linienförmige Elektrodenkörper (2) in dem bogenförmigen Bereich (10) seine Richtung um mindestens 150° ändert.

6. Elektrodenanordnung (18) nach einem der vorhergehenden Ansprüche, **dadurch gekennzeichnet, dass** sich der jeweilige linienförmige Elektrodenkörper (2) von und bis zu einem Sockel (3) aus einem elektrisch isolierenden Material erstreckt.

7. Elektrodenanordnung (18) nach einem der vorhergehenden Ansprüche, **dadurch gekennzeichnet, dass** sich der Behandlungsraum (9) zumindest teilweise zwischen zwei Ebenen befindet, in denen sich zwei einander benachbarte Elektrodenkörper (2) mit ihren bogenförmigen Bereichen (10) erstrecken.

8. Elektrodenanordnung (18) nach einem der vorhergehenden Ansprüche, **dadurch gekennzeichnet, dass** sich der Behandlungsraum (9) zumindest teilweise zwischen zwei Gruppen (14, 15) der Elektrodenkörper (2) erstreckt, deren bogenförmigen Bereiche (10) sich mit einander entgegen gerichteten Krümmungsradien gegenüber liegen.

9. Elektrodenanordnung (18) nach Anspruch 8, **dadurch gekennzeichnet, dass** die bogenförmigen Bereiche (10) der Elektrodenkörper (2) jeder Gruppe (14, 15) in zueinander parallelen Ebenen verlaufen.

10. Elektrodenanordnung (18) nach einem der vorhergehenden Ansprüche, **dadurch gekennzeichnet, dass** die Elektrodenkörper (2) in den bogenförmigen Bereichen (10) verformbar sind.

11. Elektrodenanordnung (18) nach einem der vorhergehenden Ansprüche, **dadurch gekennzeichnet, dass** die Elektrodenkörper (2) gegen elastische Kräfte und/oder Gravitationskräfte verformbar sind.

12. Elektrodenanordnung (18) nach einem der vorhergehenden Ansprüche, **dadurch gekennzeichnet, dass** die Elektrodenkörper (2) in einem den Behandlungsraum (9) teilweise umschließenden Gehäuse (16) angeordnet sind.

13. Elektrodenanordnung (18) nach Anspruch 12, **dadurch gekennzeichnet, dass** das Gehäuse (16) geerdet ist.

14. Plasmabehandlungsvorrichtung (1) mit einem Wechselhochspannungsgenerator (7), der an einem Ausgang (6) eine Wechselhochspannung gegenüber Erde oder Masse erzeugt, und einer Elektrodenanordnung (18) nach einem der vorhergehenden Ansprüche, die an den Ausgang (6) des Wechselhochspannungsgenerators (7) angeschlossen ist.

15. Plasmabehandlungsvorrichtung (1) nach Anspruch 14, **dadurch gekennzeichnet, dass** der Wechselhochspannungsgenerator (7) eine Wechselhochspannung von 5 kV bis 30 kV in Form von Hochspannungspulspaaren mit einer Pulswiederholungsfrequenz von 3 kHz bis 50 kHz und mit einer elektrischen Leistung von 5 W bis 500 W erzeugt.

## Claims

1. Electrode arrangement (18) for a surface treatment of a body (11) with a physical plasma, comprising:
- a high voltage connection (19) for connecting to an output (6) of an alternating high voltage source generating an alternating high voltage;
- several line-shaped electrode bodies (2) which are together connected to the alternating high voltage connection (19) in such a way that the same alternating high voltage is present at all electrode bodies (2), and which are covered by a dielectric (4) and/or individually capacitively coupled to the high voltage connection (19); and
- a treatment space (9) adjoining the electrode bodies (2), in which the body (11) can be introduced for the surface treatment with the physical plasma;
**characterised in that** each of the electrode bodies (2) includes an arch-shaped area (20) in which the respective line-shaped electrode body (2) changes its direction by at least 90°, wherein the treatment space (9) is at least partially located between the arch-shaped areas (10) of the electrode bodies (2).

2. Electrode arrangement (18) of claim 1, **characterised in that** a radius of curvature (10) at which the respective line-shaped electrode body (2) changes its direction in the arch-shaped area (10) is between 3 mm and 300 mm or between 5 mm and 200 mm.

3. Electrode arrangement (18) of claim 1 or 2, **characterised in that** the respective line-shaped electrode body (2) has a constant smallest radius of surface curvature (21) between 0.3 mm and 30 mm or between 0.5 mm and 5 mm in the arch-shaped area (10).

4. Electrode arrangement (18) of any of the preceding claims, **characterised in that** the electrode bodies (2) have an outer surface with a rounded or circular cross section in the arch-shaped area (10).

5. Electrode arrangement (18) of any of the preceding claims, **characterised in that** the respective line-shaped electrode body (2) changes its direction by at least 150° in the arch-shaped area (10).

6. Electrode arrangement (18) of any of the preceding claims, **characterised in that** the respective line-shaped electrode body (2) extends from and up to a socket (3) made of an electrically isolating material.

7. Electrode arrangement (18) of any of the preceding claims, **characterised in that** the treatment space (9) is at least partially located between two planes in which two neighbouring electrode bodies (2) extend with their arch-shaped areas (10).

8. Electrode arrangement (18) of any of the preceding claims, **characterised in that** the treatment space (9) at least partially extends between two groups (14, 15) of the electrode bodies (2), whose arch-shaped areas (10) are opposite to each other with opposing radiuses of curvature.

9. Electrode arrangement (18) of claim 8, **characterised in that** the arch-shaped areas (10) of the electrode bodies (2) of each group (14, 15) run in planes which are parallel to each other.

10. Electrode arrangement (18) of any of the preceding claims, **characterised in that** the electrode bodies (2) are deformable in the arch-shaped areas (10).

11. Electrode arrangement (18) of any of the preceding claims, **characterised in that** the electrode bodies (2) are deformable against elastic forces and/or gravitational forces.

12. Electrode arrangement (18) of any of the preceding claims, **characterised in that** the electrode bodies (2) are arranged in a housing (16) partially enclosing the treatment space (9).

13. Electrode arrangement (18) of claim 12, **characterised in that** the housing (16) is grounded.

14. Plasma treatment apparatus (1) comprising an alternative high voltage generator (7) generating an alternative high voltage with regard to ground or earth at an output (6), and an electrode arrangement (18) of any of the preceding claims which is connected to the output (16) of the alternating high voltage generator (7).

15. Plasma treatment apparatus (1) of claim 14, **characterised in that** the alternating high voltage generator (7) generates an alternative high voltage of 5 kV to 30 kV in form of high voltage pulse pairs having a pulse repetition frequency of 3 kHz to 50 kHz and an electric power of 5 W to 500 W.

## Revendications

1. Disposition d'électrodes (18) pour un traitement de surface d'un corps (11) avec un plasma physique, avec :
- un raccordement à haute tension (19) pour le raccordement à une sortie (6) d'une source de haute tension alternative générant une haute tension alternative ;
- plusieurs corps d'électrodes linéaires (2), qui sont raccordés ensemble au raccordement à haute tension (19) de façon à ce que, à tous les corps d'électrodes (2), la même haute tension alternative soit appliquée et qui sont enveloppés d'un diélectrique (4) et/ou qui sont couplés individuellement de manière capacitive au raccordement à haute tension (19) ; et
- un espace de traitement (9) adjacent aux corps d'électrodes (2), dans lequel le corps (11) peut être introduit pour le traitement de surface avec le plasma physique ;
**caractérisée en ce que** les corps d'électrodes (2) comprennent chacun une zone en forme d'arc de cercle (10) dans laquelle le corps d'électrode linéaire (2) correspondant change de direction d'au moins 90°, dans lequel l'espace de traitement (9) se trouve au moins partiellement entre les zones en arc de cercle (10) et le corps d'électrode (2).

2. Disposition d'électrodes (18) selon la revendication 1, **caractérisée en ce qu'**un rayon de courbure (20), avec lequel le corps d'électrode linéaire (2) correspondant change de direction dans la zone en arc de cercle (10), est entre 3 mm et 300 mm ou entre 5 mm et 200 mm.

3. Disposition d'électrodes (18) selon la revendication 1 ou 2, **caractérisée en ce que** le corps d'électrode linéaire (2) correspondant présente, dans la zone en arc de cercle (10), un rayon de courbure de surface (21) constant très faible entre 0,3 mm et 30 mm ou entre 0,5 mm et 5 mm.

4. Disposition d'électrodes (18) selon l'une des revendications précédentes, **caractérisée en ce que** les corps d'électrodes (2) présentent, dans les zones en arc de cercle (10), une surface externe avec une section transversale arrondie ou circulaire.

5. Disposition d'électrodes (18) selon l'une des revendications précédentes, **caractérisée en ce que** le corps d'électrode linéaire (2) correspondant change, dans la zone en arc de cercle (10), de direction d'au moins 150°.

6. Disposition d'électrodes (18) selon l'une des revendications précédentes, **caractérisée en ce que** le corps d'électrode linéaire (2) s'étend de et jusqu'à un socle (3) constitué d'un matériau électriquement isolant.

7. Disposition d'électrodes (18) selon l'une des revendications précédentes, **caractérisée en ce que** l'espace de traitement (9) se trouve au moins partiellement entre deux plans, dans lesquels s'étendent deux corps d'électrodes (2) adjacents avec leurs zones en arc de cercle (10).

8. Disposition d'électrodes (18) selon l'une des revendications précédentes, **caractérisée en ce que** l'espace de traitement (9) s'étend au moins partiellement entre deux groupes (14, 15) de corps d'électrodes (2), dont les zones en arc de cercle (10) avec des rayons de courbures opposés se font face.

9. Disposition d'électrodes (18) selon la revendication 8, **caractérisée en ce que** les zones en arc de cercle (10) des corps d'électrodes (2) de chaque groupe (14, 15) s'étendent dans des plans parallèles.

10. Disposition d'électrodes (18) selon l'une des revendications précédentes, **caractérisée en ce que** les corps d'électrodes (2) sont déformables dans les zones en arc de cercle (10).

11. Disposition d'électrodes (18) selon l'une des revendications précédentes, **caractérisée en ce que** les corps d'électrodes (2) sont déformables contre des forces élastiques et/ou des forces de gravité.

12. Disposition d'électrodes (18) selon l'une des revendications précédentes, **caractérisée en ce que** les corps d'électrodes (2) sont disposés dans un boîtier entourant partiellement l'espace de traitement (9).

13. Disposition d'électrodes (18) selon la revendication 12, **caractérisée en ce que** le boîtier (16) est branché à la terre.

14. Dispositif de traitement au plasma (1) avec un générateur de haute tension alternative (7) qui génère, à une sortie (6), une haute tension alternative par rapport à la terre ou à la masse et une disposition d'électrodes (18) selon l'une des revendications précédentes, qui est raccordée à la sortie (6) du générateur de haute tension alternative (7).

15. Dispositif de traitement au plasma (1) selon la revendication 14, **caractérisé en ce que** le générateur de haute tension alternative (7) génère une haute tension alternative de 5 kV à 30 kV sous la forme de paires d'impulsions de haute tension avec une fréquence de répétition d'impulsions de 3 kHz à 50 kHz et avec une puissance électrique de 5 W à 500 W.
